# EUROPEAN PATENT APPLICATION

(11) **EP 4 403 168 A1**
(43) Date of publication of application: **24.07.2024**
(21) Application number: 23152206.1
(22) Date of filing: 18.01.2023
(51) Int. Cl.: A61K 9/24, A61K 31/155, A61K 31/7048, A61P 3/10

(54) **FIXED-DOSE COMBINATION COMPOSITION OF METFORMIN AND EMPAGLIFLOZIN**

(71) Applicant: Adamed Pharma S.A., 05-152 Czosnow (PL)
(72) Inventor: BOLTROMIUK, Tomasz, 02-496 Warszawa (PL); SIELUK, Kinga, 05-230 Kobylka (PL); GAJDA, Maciej, 05-082 Stare Babice (PL)
(74) Representative: Sulikowski, Daniel

(57) **Abstract**

A fixed-dose pharmaceutical preparation in the form of a coated tablet for oral administration comprising extended release metformin hydrochloride as the core of the tablet, coated with an intermediate coating based on hydroxypropylmethylcellulose as a film-forming material, further coated with empagliflozin comprising active coating based on partially hydrolyzed polyvinyl alcohol as a film-forming material, and further optionally coated with outer protective coating.

## Description

### Field of the invention

The invention relates to a fixed-dose combination composition in the form of a tablet for oral administration comprising extended release metformin hydrochloride and immediate release empagliflozin in a single dosage unit. The tablet can find use in the treatment of type 2 diabetes mellitus.

### Prior art

The treatment of type 2 diabetes mellitus often requires combined, simultaneous administration of more than one active agents having different mechanisms of action to control the disease when treatment with a single agent fails to allow proper glycaemia control. One of such combined therapies involves combined administration of metformin hydrochloride and SGLT2 inhibitors. Metformin lowers blood glucose level and exerts its action mainly in the liver by reducing gluconeogenesis. SGLT2 inhibitors lower blood glucose level in patients with type 2 diabetes by reducing renal glucose reabsorption, this leading to secretion of excess of glucose in the urine.

One of widely used SGLT2 inhibitors is empagliflozin. Empagliflozin is administered once-daily as a conventional, immediate release tablet. Appropriate dosage form should provide release profile of empagliflozin with quick dissolution of the active substance as well as its complete dissolution (so called infinity point at the level of 100%). Thus, urinary glucose excretion increases immediately following the first dose of empagliflozin and is continuous over the 24 hour dosing interval. A product for once daily administration presenting such characteristics, containing empagliflozin as the only active agent is present on the market under the trade name Jardiance^{®}.

Metformin hydrochloride may be formulated as an extended release (ER), long-acting formulation, i.e. tablet, with longer half-life and lower peak drug concentration comparing to immediate release (IR), conventional metformin formulations. Metformin ER and IR have similar effectiveness and safety but ER formulation is associated with improved compliance to treatment as it can be taken only once-daily.

Both active agents can be administered in combination but separately, as mono products (tablets). However, it is always advisable to have combination products, so called fixed-dose products wherein both active agents are comprised in the same single dosage unit. Such fixed-dose combination products are indicated in patients already being treated with the combination of empagliflozin and metformin as separate tablets and should therefore provide similar pharmacokinetics and absorption in vivo, as respective individual substance when taken separately. In particular, dissolution characteristics of active substances should be similar.

Extended release form of metformin hydrochloride that can be taken as once-daily preparation are known. Fixed-dose combinations of extended release form of metformin hydrochloride with once-daily empagliflozin to improve patient convenience and compliance with the treatment are highly desired.

However, due to quite high dose of metformin hydrochloride, up to 1000 mg to be taken daily in a single unit dosage form, conventional combination tablets or bilayer tablets would have quite high mass, up to about 2000 mg, and would be difficult to swallow.

The problem of high mass of a single dosage unit of combined composition has been solved in the prior art by providing tablets that comprise extended release metformin hydrochloride in the tablet core, while empagliflozin is included as a component of the coating film on the core (active coating).

WO2012120040A1 discloses a fixed-dose pharmaceutical composition in the form of a tablet for oral administration in the treatment of diabetes, wherein metformin extended release tablet core is coated with an immediate release active polymeric film coating containing empagliflozin or linagliptin as the active ingredient. The inner extended release core comprises metformin hydrochloride and extended release matrix-forming polymer together with one or more excipients. The active immediate release coating with empagliflozin as the active ingredient may comprise hydroxypropylmethylcellulose as a film-forming agent, polyethylene glycol or propylene glycol as a plasticizer and a glidant (i.e. anti-tacking agent) such as talc. The composition may include an optional intermediate seal coating on top of the inner core wherein a film-forming agent may be a mixture of hydroxypropylcellulose and hydroxypropylmethylcellulose, a mixture of polyvinyl alcohol (PVA) and polyethylene glycol (PEG), or a mixture of hydroxypropyl methylcellulose and either polyethylene glycol (PEG) or propylene glycol (PG). A final outer over-coating may optionally be present on the top of the active coating.

Such type fixed-dose composition is also currently available on the market. According to FDA label, commercial preparation SynjardyXR^{®} is a tablet that comprises metformin hydrochloride extended-release core that is coated with the immediate-release empagliflozin coating and all coatings are based on hydroxypropylmethylcellulose as a film-forming agent.

Specific embodiments of the tablet formulation of WO2012120040A1 containing extended release metformin core, barrier (intermediate) seal coating and immediate release empagliflozin active coating are disclosed in WO2013131967 in Tables 3, 4A and 4B. Disclosed empagliflozin active coatings may include HPMC as the film-forming material, polyethylene glycol as the plasticizer and talc as the glidant, while barrier (i.e. intermediate) coating includes the mixture of 3 types of HPMC and polydextrose as the film-forming material (polydextrose being the main component of the film forming system), polyethylene glycol as the plasticizer and titanium dioxide as a pigment, and does not include any glidant.

In this manner the total mass of the tablet can be lower in comparison with "conventional" tablets. For example, for dosage strengths empagliflozin/metformin 12.5/1000 mg active coated tablet weighing about 1600 mg can be prepared. At the same time, preparation of a bilayer tablet of the same strength, including protective coating, would reach weight about 1900-2000 mg.

The technical problem with such type of a preparation is that empagliflozin in the coating is embedded in a film-forming polymer. Composition of the active coating layer should be designed in such a manner so as to obtain release profile similar to the immediate release tablet of empagliflozin administered separately. That is, release profile from the film of the coating should provide quick dissolution of empagliflozin, and infinity point at the level of about 100%, in particular comparable to the reference market product Jardiance^{®}. Otherwise, the combined product would not provide proper control of glycaemia, comparable to separate administration of active ingredients in single dosage units. In other words, the aim of the improvement of patient compliance should be achieved without compromising the effect of the therapy.

Generally, for immediate release formulations it is important that complete dissolution is reached before gastric emptying. For assessment, evaluation/comparison purposes dissolution at 15 min is essential criterion and should exceed 85%. Furthermore, in accordance with The European Medicines Agency recommendations for assessment and evaluation of similarity of the products, when >85% of the drug is dissolved within 15 min, dissolution profiles may be accepted as similar without further mathematical evaluation.

Furthermore, composition of the active coating and intermediate coating should also ensure appropriate chemical stability of the active substance, in accordance with requirements for pharmaceutical products.

These problems are solved by the present invention.

### Summary of the invention

The present invention provides a fixed-dose pharmaceutical composition for oral administration comprising extended-release metformin hydrochloride and immediate-release empagliflozin in a single dosage unit. The pharmaceutical composition of the invention is a coated tablet, wherein extended-release metformin hydrochloride formulation is a core of the tablet and empagliflozin is present in a coating of the tablet.

According to the invention, the composition is a coated tablet comprising :
a) an inner extended release tablet core comprising metformin hydrochloride; an extended release polymer; and one or more excipients;
b) an intermediate coating on top of the inner tablet core, comprising a film-forming material which is hydroxypropylmethylcellulose; and optionally one or two excipients selected from the group consisting of a plasticizer and an anti-tacking material;
c) an active coating on the top of the intermediate coating, comprising empagliflozin; a film-forming material which is partially hydrolyzed polyvinyl alcohol; and optionally one or two excipients selected from the group consisting of a plasticizer and an anti-tacking material; and
d) optionally, a protective coating on the top of the active coating.

### Detailed description of the invention

The problem solved by the invention is to provide low-mass, chemically stable composition of metformin hydrochloride extended release/empagliflozin intermediate release fixed-dose preparation, wherein empagliflozin dissolves quickly (i.e. dissolution at 15 min is > 85%) and completely, similarly as in the case of empagliflozin mono product.

Surprisingly, it has been found that such a desired empagliflozin release profile from metformin hydrochloride/empagliflozin fixed-dose composition could be obtained for the composition in the form of a tablet, having extended release metformin hydrochloride core tablet, intermediate inert film coating and active film coating loaded with empagliflozin when specific film-forming polymers were used for intermediate coating and active coating, i.e. hydroxypropylmethylcellulose for intermediate coating and partially hydrolyzed polyvinyl alcohol for active coating, respectively.

The present inventors have found as well that desired empagliflozin release profile of the metformin hydrochloride/empagliflozin coated tablet could not be obtained when hydroxypropylmethylcellulose was used as a film-forming polymer for both intermediate (inert) coating and the active coating comprising empagliflozin.

Therefore, the object of the invention is a fixed-dose pharmaceutical composition in the form of a coated tablet for oral administration, comprising extended release metformin hydrochloride and immediate release empagliflozin, comprising:
a) an inner extended release tablet core comprising metformin hydrochloride; an extended release polymer; and one or more excipients;
b) an intermediate coating on top of the inner tablet core, comprising a film-forming material which is hydroxypropylmethylcellulose; and optionally one or two excipients selected from the group consisting of a plasticizer and an anti-tacking material;
c) an active coating on top the intermediate coating, comprising a film-forming material which is partially hydrolyzed polyvinyl alcohol; empagliflozin; and optionally one or two excipients selected from the group consisting of a plasticizer and an anti-tacking material; and
d) optionally, a protective coating on the top of the active coating.

It will be appreciated by a person skilled in the art that any one and all coatings in the pharmaceutical composition is an immediate release coating.

The invention in all its embodiments and variants described herein encompasses also the fixed-dose pharmaceutical composition in the form of a coated tablet for oral administration, comprising extended release metformin hydrochloride and immediate release empagliflozin, consisting of:
a) an inner extended release tablet core comprising metformin hydrochloride; an extended release polymer; and one or more excipients;
b) an intermediate coating on top of the inner tablet core, comprising a film-forming material which is hydroxypropylmethylcellulose; and optionally one or two excipients selected from the group consisting of a plasticizer and an anti-tacking material;
c) an active coating on top the intermediate coating, comprising a film-forming material which is partially hydrolyzed polyvinyl alcohol; empagliflozin; and optionally one or two excipients selected from the group consisting of a plasticizer and an anti-tacking material; and
d) optionally, a protective coating on the top of the active coating.

Additionally, the present inventors have found as well that chemical stability of the tablet of the invention may be improved when active coating or both intermediate and active coatings b) and c), respectively, comprise a sugar as a stabilizer.

Therefore, in a specific and preferred embodiment, active coating c) further comprises a sugar as a stabilizer.

In another specific and preferred embodiment, intermediate coating b) further comprises a sugar as a stabilizer. Specifically, both intermediate b) and active coating c) comprise a sugar as the stabilizer.

Therefore, in a specific embodiment the object of the invention is the fixed-dose pharmaceutical composition in the form of a coated tablet for oral administration, comprising extended release metformin hydrochloride and immediate release empagliflozin, comprising:
a) the inner extended release tablet core comprising metformin hydrochloride; the extended release polymer; and one or more excipients;
b) the intermediate coating on top of the inner tablet core, comprising the film-forming material which is hydroxypropylmethylcellulose; and optionally one or two excipients selected from the group consisting of the plasticizer and the anti-tacking material;
c) the active coating on top the intermediate coating, comprising the film-forming material which is partially hydrolyzed polyvinyl alcohol; empagliflozin; the sugar as a stabilizer; and optionally one or two excipients selected from the group consisting of the plasticizer and the anti-tacking material; and
d) optionally, the protective coating on the top of the active coating.

In another specific embodiment the object of the invention is the fixed-dose pharmaceutical composition in the form of a coated tablet for oral administration, comprising extended release metformin hydrochloride and immediate release empagliflozin, comprising:
a) the inner extended release tablet core comprising metformin hydrochloride; the extended release polymer and one or more excipients;
b) the intermediate coating on top of the inner tablet core, comprising the film-forming material which is hydroxypropylmethylcellulose; the sugar as a stabilizer; and optionally one or two excipients selected from the group consisting of the plasticizer and the anti-tacking material;
c) the active coating on top the intermediate coating, comprising the film-forming material which is of partially hydrolyzed polyvinyl alcohol; empagliflozin; the sugar as a stabilizer; and optionally one or two excipients selected from the group consisting of the plasticizer and anti-tacking material; and
d) optionally, the protective coating on the top of the active coating.

Metformin hydrochloride and empagliflozin may be present in the coated tablet of the invention according to various embodiments described above in various dosage strengths, which are known in the art, corresponding to dosage strengths in respective mono-products and to the required treatment schemes.

Specifically, metformin hydrochloride may be present in the preparation of the invention in a unit dosage strength of 500, 750, 850, 1000 or 1500 mg, especially 750 or 1000 mg, per unit tablet.

Specifically, empagliflozin may be present in the preparation of the invention in a unit dosage strength of 2.5, 5, 10, 12.5 or 25 mg, especially 5 or 12.5 mg, per unit tablet.

Specific combination empagliflozin/metformin (mg) dosage strengths are 5/750 mg, 12.5/750 mg, 5/1000 mg and 12.5/1000 mg, per unit tablet.

The active coating c) comprises empagliflozin, i.e. the active coating c) is empagliflozin-loaded. In any of the embodiments described above, empagliflozin is present in the amount in the range from 5 to 20% by weight with respect to the weight of the active coating c), depending on the specific dosage strength and the composition of the tablet.

The active coating c) comprises partially hydrolyzed polyvinyl alcohol (also known under acronym PVA) as a film-forming material. The amount of partially hydrolyzed polyvinyl alcohol in the active coating c) as described above is in the range from 20 to 60% by weight with respect to the weight of the active coating c).

Partially hydrolyzed poly vinyl alcohol has degree of hydrolysis below 98 mol%, such as degree of hydrolysis of 85-96 mol%), in particular degree of hydrolysis between 86.5 mol% and 90 mol%, especially degree of hydrolysis between 86.5 to 89.0 mol%.

As described above, the active coating c), the intermediate coating b) or both the active coating c) and the intermediate coating b) may further comprise the sugar as a stabilizer.

Sugar to be used in the active coating c) and in the intermediate coating b) in any of the embodiments described above can be any sugar suitable and acceptable for use in pharmaceutical products, in particular monosaccharide, disaccharide and polysaccharide. Thus, sugar may be especially selected from the group consisting of mannitol, sorbitol, isomalt, lactose, and polydextrose. Preferred sugar is polydextrose.

When both coatings b) and c) comprise a sugar, the sugar in the active coating b) may be the same or different than the sugar in the intermediate coating c). Preferably, when both coatings b) and c) comprise a sugar, the sugar in the intermediate coating b) is the same as the sugar in the active coating c), in such a case the most preferred sugar in both coatings is polydextrose.

The amount of sugar, in particular polydextrose, in the intermediate coating b) may be in a range from 0 to 30%, especially from 2 to 30%, by weight with respect to the weight of the intermediate coating b).

The amount of sugar, in particular polydextrose, in the active coating c) may be in a range from 0 to 30%, especially from 2 to 30%, by weight with respect to the weight of the active coating c).

Sugar may play important role when partially hydrolyzed polyvinyl alcohol is used as a film-forming material for empagliflozin containing coatings. The use of partially hydrolyzed polyvinyl alcohol, containing non-hydrolyzed acetyl groups may potentially jeopardize stability of empagliflozin due to the risk of acetylation of empagliflozin hydroxyl groups. However, it has been found unexpectedly that the presence of the sugar in the coating or coatings diminishes to high extent such acetylation reaction and improves chemical stability of empagliflozin in the composition.

When polydextrose is used as a stabilizing sugar, it may exert additional effect of enhancing film adhesion and improving mechanical properties of the tablets (i.e. it may act as an adhesion modifier).

The intermediate coating b) between the core and the active coating c) comprises hydroxypropylmethylcellulose (also known under the name hypromellose or HPMC) as the film-forming material. The amount of hydroxypropylmethylcellulose is in the range from 60 to 98% by weight with respect to the weight of the intermediate coating b).

Hydroxypropylmethylcellulose types that can be used as a material for the intermediate coating b) include those with viscosities in the range 1 to 100 MPas, preferably 3 to 15 MPas. For example, suitable and preferred material is HPMC E type (substitution type 2910), such as low-molecular weight HPMC E3 or E6.

In one embodiment, the active coating c) or the intermediate coating b) or both the active coating c) and the intermediate coating b) may further comprise the plasticizer.

In another embodiment, The intermediate coating b) or active coating c), or both the intermediate coating b) and the active coating c) may further comprise the optional anti-tacking agent.

In yet another embodiment, the intermediate coating b) or active coating c), or both the intermediate coating b) and the active coating c) may further comprise both the plasticizer and the anti-tacking material.

Preferred plasticizer for the active coating c) is propylene glycol, glycerin, or polyethylene glycol, especially polyethylene glycol of molecular weight in the range 400 to 8000, such as polyethylene glycol (Macrogol) 3350, 4000, 6000 or 8000, especially polyethylene glycol 3350 (Macrogol 3350).

The amount of plasticizer in the active coating c) is in the range from 5 to 35% by weight with respect to the weight of the active coating c) and will depend on the amount of film-forming material and insoluble matter.

Preferred plasticizer for the intermediate coating b) is propylene glycol, glycerin, or polyethylene glycol, especially polyethylene glycol of molecular weight in the range 400 to 8000, such as polyethylene glycol (Macrogol) 4000, 6000 or 8000, especially polyethylene glycol 8000 (Macrogol 8000).

The amount of plasticizer in the intermediate coating b) is in the range from 1 to 5% by weight with respect to the weight of the intermediate coating b) and will depend on the amount of film-forming material and insoluble matter.

Although various anti-tacking materials suitable for coatings can be used for both the intermediate coating b) and the active coating c), such as calcium phosphate, calcium silicate, cellulose, colloidal silicon dioxide, hydrophobic colloidal silica, magnesium oxide, magnesium silicate, magnesium trisilicate, silicon dioxide, and talc, preferred anti-tacking material for both the intermediate coating b) and the active coating c) is talc.

The amount of the anti-tacking material, preferably talc, is in the range from 1 to 5% by weight with respect to the weight of the active coating c) and in the range from 1 to 5% by weight with respect to the weight of the intermediate coating b), and may depend on the characteristics of the film forming polymer.

In another specific embodiment, the intermediate coating b) consists of hydroxypropylmethylcellulose, polyethylene glycol 8000, and talc.

In another specific embodiment, the intermediate coating b) consists of hydroxypropylmethylcellulose, polyethylene glycol, especially polyethylene glycol 8000, polydextrose and talc.

In another specific embodiment, the active coating c) consists of partially hydrolyzed polyvinyl alcohol, empagliflozin, polyethylene glycol, especially polyethylene glycol 3350, and talc.

In another specific embodiment, the active coating c) consists of partially hydrolyzed polyvinyl alcohol, empagliflozin, polyethylene glycol, especially polyethylene glycol 3350, polydextrose and talc.

In yet another specific embodiment the intermediate coating b) consists of hydroxypropylmethylcellulose, polyethylene glycol, especially polyethylene glycol 8000, polydextrose and talc, and the active coating c) consists of partially hydrolyzed polyvinyl alcohol, empagliflozin, polyethylene glycol, especially polyethylene glycol 3350, polydextrose and talc.

In yet another specific embodiment the intermediate coating b) consists of hydroxypropylmethylcellulose, polyethylene glycol, especially polyethylene glycol 8000, and talc, and the active coating c) consists of partially hydrolyzed polyvinyl alcohol, empagliflozin, polyethylene glycol, especially polyethylene glycol 3350, polydextrose and talc.

In yet another specific embodiment the intermediate coating b) consists of hydroxypropylmethylcellulose, polyethylene glycol, especially polyethylene glycol 8000, and talc, and the active coating c) consists of partially hydrolyzed polyvinyl alcohol, empagliflozin, polyethylene glycol, especially polyethylene glycol 3350, and talc.

Use of specific film-forming materials in respective coatings allows to solve the problem underlying the present invention, i.e. providing desired release profile of empagliflozin. As it will be shown further below, desired release profile could not have been obtained when hydroxypropylmethylcellulose was used as a film-forming material in both coatings. Desired release profile could also not have been obtained when partially hydrolyzed polyvinyl alcohol was used as a film-forming material in both coatings.

The inner extended release tablet core a) of the composition of the invention comprises metformin hydrochloride. The inner tablet core is a formulation wherein metformin hydrochloride is dispersed and embedded in an extended release polymer as a matrix forming material and compressed into tablet form. Such extended release matrix metformin hydrochloride formulations are conventional and known, for example from WO2012120040A1 and references cited therein, WO2011060256 and WO2013131967.

Extended release polymer (i.e. matrix-forming material) is a hydrophilic polymer that is water swellable and may be selected from the group consisting of hydroxypropylmethylcellulose polymers, polyethylene oxide) polymers, alginic acid, sodium and calcium alginates, xanthan gum, and sodium carboxymethylcellulose, as well as their mixtures.

Preferably, extended release polymer (i.e. matrix-forming material) is hydroxypropylmethylcellulose (HPMC). A person skilled in the art will know which types of hydroxypropylmethylcellulose can be used as matrix forming materials. For example, suitable and preferred material is HPMC K type (substitution type 2208), such as HPMC K100M.

The inner tablet core a) further comprises one or more excipients.

Said one or more excipients may be a lubricant. Preferred lubricant is magnesium stearate.

Said one or more excipients in the inner tablet core a) may be a binder (granulation-aid), which preferably is low-molecular weight, low-viscosity hydroxypropylmethylcellulose. A person skilled in the art will know which types of hydroxypropylmethylcellulose can be used as binders. For example, suitable and preferred material is HPMC E type (substitution type 2910), such as low-molecular weight HPMC E6 or E50.

In a specific embodiment, said one or more excipients in the inner tablet core a) are the lubricant and the binder. In the preferred embodiment, said one or more excipients are magnesium stearate as the lubricant and hydroxypropylmethylcellulose as the binder.

In a preferred variant of any one of the above embodiments, the composition comprises the protective coating d).

Preferably, the protective coating d) is a standard polyvinyl alcohol based coating, especially colored/pigmented standard polyvinyl alcohol based coating, such as for example Opadry polyvinyl alcohol based coating. Colors may be used to differentiate various tablet strengths.

In one specific embodiment, the preparation of the invention comprises:
a) the inner extended release tablet core consisting of metformin hydrochloride, hydroxypropylmethylcellulose as the extended release polymer, low-molecular hydroxypropylmethylcellulose as the binder, and magnesium stearate as the lubricant;
b) the intermediate coating on top of the inner tablet core, consisting of hydroxypropylmethylcellulose; polyethylene glycol 8000; and talc;
c) the active coating on top the intermediate coating, consisting of partially hydrolyzed polyvinyl alcohol; empagliflozin; polydextrose; polyethylene glycol 3350; and talc;
   and
d) polyvinyl alcohol based protective coating on the top of the active coating.

In another specific embodiment, the preparation of the invention comprises:
a) the inner extended release tablet core consisting of metformin hydrochloride, hydroxypropylmethylcellulose as the extended release polymer, low-molecular hydroxypropylmethylcellulose as the binder, and magnesium stearate as the lubricant;
b) the intermediate coating on top of the inner tablet core, consisting of hydroxypropylmethylcellulose; polyethylene glycol 8000; polydextrose and talc;
c) the active coating on top the intermediate coating, consisting of partially hydrolyzed polyvinyl alcohol; empagliflozin; polydextrose; polyethylene glycol 3350; and talc;
   and
d) polyvinyl alcohol based protective coating on the top of the active coating.

The inner extended release tablet core may be prepared in a manner well known for a person skilled in the art, by compressing suitable solid formulation (tableting mass/blend) into a tablet. Suitable solid tableting mass/blend may be prepared in a known manner by granulation of metformin hydrochloride in a wet granulation process (high shear granulation or fluid bed granulation). Solid blend of metformin hydrochloride with the binder or part of the binder, and pure water or an aqueous solution of the remaining part of the binder, respectively, as a granulation liquid may be used for the granulation process. Granulate thus obtained is dried and screened, and the matrix-forming polymer and the lubricant are added extragranularly to obtain tableting mass.

Tableting mass/blend for the inner tablet core may be prepared without a binder. Since metformin hydrochloride is very well soluble in water and matrix forming polymer is a high viscosity one, wet granulation may be performed with pure water in a fluid bed granulator when proper bed temperature and air flow are used to obtain granulate with intragranular matrix-forming polymer. Granulate thus obtained, after drying and sieving, is added with a lubricant extragranularly to obtain tableting mass.

Coatings may be applied in a manner well known for a person skilled in the art, for example by spraying, aqueous solutions of a blend of coating components onto the cores.

### Example 1

General procedure for the preparation of extended release metformin hydrochloride formulation/immediate release empagliflozin coated tablets

The core of the coated tablet, i.e. extended release metformin hydrochloride formulation was prepared by wet-granulation tableting method. Metformin hydrochloride and the part of a binder (HPMC E50) were blended/mixed in a mixing bowl, an aqueous solution of the rest of the binder HPMC E50 was added to the powder blend and the whole mass was granulated. Wet granulation was performed in a high-shear granulator. Wet granulate thus obtained was dried in a fluid-bed drier and screened to obtain dry granulate. To the dry granulate matrix-forming polymer (HPMC K100M) and then magnesium stearate powders were consecutively separately added, with mixing after each addition, and thus obtained blend was compressed into a tablet.

The cores of the tablets were coated with an aqueous suspension of intermediate sub-coating composition to the weight gain of 3% and then with an aqueous suspension of empagliflozin-comprising active coating composition to the weight gain of 4.5% plus empagliflozin dose.

Finally, tablets coated with empagliflozin active layer obtained as above were coated with final aesthetic/cosmetic color protective coating using an aqueous suspension of standard commercial Opadry PVA color coating to the weight gain of about 2 - 3%. Different colors were used for different strengths of the tablets.

Applying the above general procedure, tablets comprising 12.5 mg of empagliflozin and 1000 mg of metformin hydrochloride in a unit dosage form (strength 12.5/1000 mg) were obtained. The compositions of the cores, an intermediate sub-coating and active coating per unit dosage are presented in Tables 1A to 1E below. The following abbreviations are used;
PVA - poly vinyl alcohol
HPMC: hydroxypropylmethylcellulose
Macrogol: polyethylene glycol

**Table 1A. Composition of coated tablets (MEM1A)**

| Component | Function | Weight mg (% w/w) | % w/w of total tablet |
|---|---|---|---|
| Tablet core composition | | | |
| Metformin hydrochloride | Active agent | 1000.00 (68.97%) | 62.32% |
| HPMC K100M | Extended release - matrix forming polymeric material | 393.00 (27.1 %) | 24.49% |
| HPMC E50 | Binder | 50.00 (3.45%) | 3.12% |
| Magnesium stearate | Lubricant | 7.00 (0.48%) | 0.44% |
| | Sub-total | 1450.00 (100%) | |

| Intermediate coating composition | | | |
|---|---|---|---|
| HPMC 6m Pas | Film-forming material | 25.75 (59.2%) | 1.60% |
| HPMC 3m Pas | Film-forming material | 8.35 (19.20%) | 0.52% |
| Polydextrose | Stabilizer/adhesion modifier | 6.52 (15.00%) | 0.41% |
| Macrogol 8000 | Plasticizer | 1.57 (3.60%) | 0.10% |
| Talc | Anti-tacking agent | 1.31 (3.00%) | 0.08% |
| | Sub-total | 43.50 (100%) (weight gain 3%) | |

| Active coating composition | | | |
|---|---|---|---|
| Empagliflozin | Active substance | 12.50 (15.68%) | 0.78% |
| PVA partially hydrolyzed | Film-forming material | 30.23 (37.99%) | 1.89% |
| Polydextrose | Stabiliser/adhesion modifier | 10.08 (12.65%) | 0.63% |
| Macrogol 3350 | Plasticizer | 15.42 (19.35 %) | 0.96% |
| Talc | Anti-tacking agent | 11.43 (14.33%) | 0.71% |
| | Sub-total | 79.71 (100%) (weight gain 4.5% + 12.50 mg) | |
| | | | |
| Aesthetic pigmented Opadry PVA based protective coating | | 31.46 (weight gain 2%) | 1.96% |
| Tablet total | | 1604.67 | |

**Table 1 B: Composition of coated tablets (MEM1B) - no plasticizer in the intermediate coating**

| Component | Function | Weight mg (% w/w) | % w/w of total tablet |
|---|---|---|---|
| Tablet core composition | | | |
| Metformin hydrochloride | Active agent | 1000.00 (68.97%) | 62.32% |
| HPMC K100M | Extender release - matrix forming polymeric material | 393.00 (27.1 %) | 24.49% |
| HPMC E50 | Binder | 50.00 (3.45%) | 3.12% |
| Magnesium stearate | Lubricant | 7.00 (0.48%) | 0.44% |
| | Sub-total | 1450.00 (100%) | |

| Intermediate coating composition | | | |
|---|---|---|---|
| HPMC 6m Pas | Film-forming material | 27.32 (62.80%) | 1.70% |
| HPMC 3m Pas | Film-forming material | 8.35 (19.20%) | 0.52% |
| Polydextrose | Stabilizer/adhesion modifier | 6.52 (15.00%) | 0.41% |
| Talc | Anti-tacking agent | 1.31 (3.00%) | 0.08% |
| | Sub-total | 43.50 (100%) (weight gain 3%) | |

| Active coating composition | | | |
|---|---|---|---|
| Empagliflozin | Active substance | 12.50 (15.68%) | 0.78% |
| PVA partially hydrolyzed | Film-forming material | 30.23 (37.99%) | 1.89% |
| Polydextrose | Stabilizer/adhesion modifier | 10.08 (12.65%) | 0.63% |
| Macrogol 3350 | Plasticizer | 15.42 (19.35 %) | 0.96% |
| Talc | Anti-tacking agent | 11.43 (14.33%) | 0.71% |
| | Sub-total | 79.71 (100%) (weight gain 4.5% + 12.50 mg) | |
| | | | |
| Aesthetic pigmented Opadry PVA based protective coating | | 31.46 (weight gain 2%) | 1.96% |
| Tablet total | | 1604.67 | |

**Table 1C: Composition of coated tablets (MEM1C) - no anti-tacking agent in the intermediate coating**

| Component | Function | Weight mg (% w/w) | % w/w of total tablet |
|---|---|---|---|
| Tablet core composition | | | |
| Metformin hydrochloride | Active agent | 1000.00 (68.97%) | 62.32% |
| HPMC K100M | Extender release - matrix forming polymeric material | 393.00 (27.1 %) | 24.49% |
| HPMC E50 | Binder | 50.00 (3.45%) | 3.12% |
| Magnesium stearate | Lubricant | 7.00 (0.48%) | 0.44% |
| | Sub-total | 1450.00 | |

| Intermediate coating composition | | | |
|---|---|---|---|
| HPMC 6m Pas | Film-forming material | 27.06 (62.20%) | 1.68% |
| HPMC 3m Pas | Film-forming material | 8.35 (19.20%) | 0.52% |
| Polydextrose | Stabilizer/adhesion modifier | 6.52 (15.00%) | 0.41% |
| Macrogol 8000 | Plasticizer | 1.57 (3.60%) | 0.10% |
| | Sub-total | 43.50 (weight gain 3%) | |

| Active coating composition | | | |
|---|---|---|---|
| Empagliflozin | Active substance | 12.50 (15.68%) | 0.78% |
| PVA partially hydrolyzed | Film-forming material | 30.23 (37.99%) | 1.89% |
| Polydextrose | Stabilizer/adhesion modifier | 10.08 (12.65%) | 0.63% |
| Macrogol 3350 | Plasticizer | 15.42 (19.35 %) | 0.96% |
| Talc | Anti-tacking agent | 11.43 (14.33%) | 0.71% |
| | Sub-total | 79.71 (100%) (weight gain 4.5% + 12.50 mg) | |
| | | | |
| Aesthetic pigmented Opadry PVA based protective coating | | 31.46 (weight gain 2%) | 1.96% |
| Tablet total | | 1604.67 | |

**Table 1D: Composition of coated tablets (MEM1D) - no plasticizer in the active coating**

| Component | Function | Weight mg (% w/w) | % w/w of total tablet |
|---|---|---|---|
| Tablet core composition | | | |
| Metformin hydrochloride | Active agent | 1000.00 (68.97%) | 62.32% |
| HPMC K100M | Extender release - matrix forming polymeric material | 393.00 (27.1 %) | 24.49% |
| HPMC E50 | Binder | 50.00 (3.45%) | 3.12% |
| Magnesium stearate | Lubricant | 7.00 (0.48%) | 0.44% |
| | Sub-total | 1450.00 (100%) | |

| Intermediate coating composition | | | |
|---|---|---|---|
| HPMC 6m Pas | Film-forming material | 25.75 (59.2%) | 1.60% |
| HPMC 3m Pas | Film-forming material | 8.35 (19.20%) | 0.52% |
| Polydextrose | Stabilizer/adhesion modifier | 6.52 (15.00%) | 0.41% |
| Macrogol 8000 | Plasticizer | 1.57 (3.60%) | 0.10% |
| Talc | Anti-tacking agent | 1.31 (3.00%) | 0.08% |
| | Sub-total | 43.50 (100%) (weight gain 3%) | |

| Active coating composition | | | |
|---|---|---|---|
| Empagliflozin | Active substance | 12.50 (15.68%) | 0.78% |
| PVA partially hydrolyzed | Film-forming material | 45.65 (57.34%) | 2.85% |
| Polydextrose | Stabilizer/adhesion modifier | 10.08 (12.65%) | 0.63% |
| Talc | Anti-tacking agent | 11.43 (14.33%) | 0.71% |
| | Sub-total | 79.71 (100%) (weight gain 4.5% + 12.50 mg) | |
| | | | |
| Aesthetic pigmented Opadry PVA based protective coating | | 31.46 (weight gain 2%) | 1.96% |
| Tablet total | | 1604.67 | |

**Table 1E. Composition of coated tablets (MEM1E) - no anti-tacking agent in the active coating**

| Component | Function | Weight mg (% w/w) | % w/w of total tablet |
|---|---|---|---|
| Tablet core composition | | | |
| Metformin hydrochloride | Active agent | 1000.00 (68.97%) | 62.32% |
| HPMC K100M | Extender release - matrix forming polymeric material | 393.00 (27.1 %) | 24.49% |
| HPMC E50 | Binder | 50.00 (3.45%) | 3.12% |
| Magnesium stearate | Lubricant | 7.00 (0.48%) | 0.44% |
| | Sub-total | 1450.00 (100%) | |

| Intermediate coating composition | | | |
|---|---|---|---|
| HPMC 6m Pas | Film-forming material | 25.75 (59.2%) | 1.60% |
| HPMC 3m Pas | Film-forming material | 8.35 (19.20%) | 0.52% |
| Polydextrose | Stabilizer/adhesion modifier | 6.52 (15.00%) | 0.41% |
| Macrogol 8000 | Plasticizer | 1.57 (3.60%) | 0.10% |
| Talc | Anti-tacking agent | 1.31 (3.00%) | 0.08% |
| | Sub-total | 43.50 (100%) (weight gain 3%) | |

| Active coating composition | | | |
|---|---|---|---|
| Empagliflozin | Active substance | 12.50 (15.68%) | 0.78% |
| PVA partially hydrolyzed | Film-forming material | 41.66 (52.32%) | 2.60% |
| Polydextrose | Stabilizer/adhesion modifier | 10.08 (12.65%) | 0.63% |
| Macrogol 3350 | Plasticizer | 15.42 (19.35 %) | 0.96% |
| | Sub-total | 79.71 (100%) (weight gain 4.5% + 12.50 mg) | |
| | | | |
| Aesthetic pigmented Opadry PVA based protective coating | | 31.46 (weight gain 2%) | 1.96% |
| Tablet total | | 1604.67 | |

### Example 2

Preparation of coated tablets of the invention having various dosage strengths

Following the procedure of Example 1 and using suitable amounts of the components, tablets having dosage strength 5/1000 mg, 12.5/750 mg and 5/750 mg were prepared. Compositions of the tablets are presented in Tables 2A, 2B and 2C, respectively.

**Table 2A. Composition of coated tablets of dosage strength 5/1000 mg (MEM2A)**

| Component | Function | Weight mg (% w/w) | % w/w of total tablet |
|---|---|---|---|
| Tablet core composition | | | |
| Metformin hydrochloride | Active agent | 1000.00 (68.97%) | 62.32% |
| HPMC K100M | Extender release - matrix forming polymeric material | 393.00 (27.1 %) | 24.49% |
| HPMC E50 | Binder | 50.00 (3.45%) | 3.12% |
| Magnesium stearate | Lubricant | 7.00 (0.48%) | 0.44% |
| | Sub-total | 1450.00 (100%) | |

| Intermediate coating composition | | | |
|---|---|---|---|
| HPMC 6m Pas | Film-forming material | 25.75 (59.2%) | 1.60% |
| HPMC 3m Pas | Film-forming material | 8.35 (19.20%) | 0.52% |
| Polydextrose | Stabilizer/adhesion modifier | 6.53 (15.00%) | 0.41% |
| Macrogol 8000 | Plasticizer | 1.57 (3.60%) | 0.10% |
| Talc | Anti-tacking agent | 1.31 (3.00%) | 0.08% |
| | Sub-total | 43.50 (100%) (weight gain 3%) | |

| Active coating composition | | | |
|---|---|---|---|
| Empagliflozin | Active substance | 5.00 (6.27%) | 0.31% |
| PVA partially hydrolyzed | Film-forming material | 30.28 (37.99%) | 1.89% |
| Polydextrose | Stabilizer/adhesion modifier | 17.58 (22.06%) | 1.10% |
| Macrogol 3350 | Plasticizer | 15.42 (19.35 %) | 0.96% |
| Talc | Anti-tacking agent | 11.43 (14.33%) | 0.71% |
| | Sub-total | 79.71 (100%) (weight gain 4.5% + 5.00 mg) | |
| | | | |
| Aesthetic pigmented Opadry PVA based coating | | 31.46 (weight gain 2%) | 1.96% |
| Tablet total | | 1604.67 | |

**Table 2B. Composition of coated tablets of dosage strength 12.5/750 mg (MEM2B)**

| Component | Function | Weight mg (% w/w) | % w/w of total tablet |
|---|---|---|---|
| Tablet core composition | | | |
| Metformin hydrochloride | Active agent | 750.00 (68.97%) | 62.32% |
| HPMC K100M | Extender release - matrix forming polymeric material | 294.75 (27.1 %) | 24.49% |
| HPMC E50 | Binder | 37.50 (3.45%) | 3.12% |
| Magnesium stearate | Lubricant | 5.25 (0.48%) | 0.44% |
| | Sub-total | 1087.50 (100%) | |

| Intermediate coating composition | | | |
|---|---|---|---|
| HPMC 6m Pas | Film-forming material | 19.31 (59.2%) | 1.60% |
| HPMC 3m Pas | Film-forming material | 6.26 (19.20%) | 0.52% |
| Polydextrose | Stabilizer/adhesion modifier | 4.89 (15.00%) | 0.41% |
| Macrogol 8000 | Plasticizer | 1.17 (3.60%) | 0.10% |
| Talc | Anti-tacking agent | 0.98 (3.00%) | 0.08% |
| | Sub-total | 32.63 (100%) (weight gain 3%) | |

| Active coating composition | | | |
|---|---|---|---|
| Empagliflozin | Active substance | 12.50 (20.91 %) | 1.04% |
| PVA partially hydrolyzed | Film-forming material | 22.70 (37.99%) | 1.89% |
| Polydextrose | Stabilizer/adhesion modifier | 4.44 (6.69%) | 0.37% |
| Macrogol 3350 | Plasticizer | 11.57 (19.35%) | 0.96% |
| Talc | Anti-tacking agent | 8.57 (14.22%) | 0.71% |
| | Sub-total | 59.78 (100%) (weight gain 4.5% + 5.00 mg) | |
| | | | |
| Aesthetic pigmented Opadry PVA based coating | | 23.60 (weight gain 2%) | 1.96% |
| Tablet total | | 1203.50 | |

**Table 2C. Composition of coated tablets of dosage strength 5/750 mg (MEM2C)**

| Component | Function | Weight mg (% w/w) | % w/w of total tablet |
|---|---|---|---|
| Tablet core composition | | | |
| Metformin hydrochloride | Active agent | 750.00 (68.97%) | 62.32% |
| HPMC K100M | Extender release - matrix forming polymeric material | 294.75 (27.1 %) | 24.49% |
| HPMC E50 | Binder | 37.50 (3.45%) | 3.12% |
| Magnesium stearate | Lubricant | 5.25 (0.48%) | 0.44% |
| | Sub-total | 1087.50 (100%) | |

| Intermediate coating composition | | | |
|---|---|---|---|
| HPMC 6m Pas | Film-forming material | 19.31 (59.2%) | 1.60% |
| HPMC 3m Pas | Film-forming material | 6.26 (19.20%) | 0.52% |
| Polydextrose | Stabilizer/adhesion modifier | 4.90 (15.00%) | 0.41% |
| Macrogol 8000 | Plasticizer | 1.17 (3.60%) | 0.10% |
| Talc | Anti-tacking agent | 0.98 (3.00%) | 0.08% |
| | Sub-total | 32.63 (100%) (weight gain 3%) | |

| Active coating composition | | | |
|---|---|---|---|
| Empagliflozin | Active substance | 5.00 (8.36%) | 0.42% |
| PVA partially hydrolyzed | Film-forming material | 22.70 (37.99%) | 1.89% |
| Polydextrose | Stabilizer/adhesion modifier | 11.94 (19.97%) | 0.99% |
| Macrogol 3350 | Plasticizer | 11.57 (19.35%) | 0.96% |
| Talc | Anti-tacking agent | 8.57 (14.22%) | 0.71% |
| | Sub-total | 59.78 (100%) (weight gain 4.5% + 5.00 mg) | |
| | | | |
| Aesthetic pigmented Opadry PVA based coating | | 23.5981 (weight gain 2%) | 1.96% |
| Tablet total | | 1203.50 | |

### Example 3

Comparative 12.5/1000 mg tablets having the same film-forming agents in intermediate and active coatings.

Comparative tablets were prepared with metformin hydrochloride 1000 mg cores of the composition as presented in Example 1 for tablets MEM1A. Compositions of intermediate and active coatings (comprising empagliflozin in the amount of 12.5 mg) are presented below in Table 3. Tablets were coated with 31.46 mg (1.96% w/w) of Opadry PVA based color standard commercial coating.

Comparative tablets MEM01, MEM13, MEM16, MEM17 and MEM20 have hydroxypropylmethylcellulose as a sole film-forming agent (polymer) in both intermediate and active coatings. Additionally, MEM20 comprised croscarmellose sodium as disintegrant in both coatings.

Comparative tablets MEM18 and MEM19 have partially hydrolyzed polyvinyl alcohol as a sole film-forming agent (polymer) in both intermediate and active coatings. MEM18 comprised additionally polydextrose as pore-forming agent, and MEM19 comprised additionally other very well soluble film forming agent HPC (hydroxypropylcellulose).

**Table 3. Compositions of comparative coated tablets 12.5/1000 mg with the same film-forming agent in intermediate and active coatings (% given as % w/w with respect to the total tablet).**

| Layer | Component | MEM01 | | MEM13 | | MEM16 | | MEM17 | | MEM18 | | MEM19 | | MEM20 | |
|---|---|---|---|---|---|---|---|---|---|---|---|---|---|---|---|
| | | mg | % | mg | % | mg | % | mg | % | mg | % | mg | % | mg | % |
| Intermediate coating | HPMC 6mPas | | | 13.92 | 0.87 | 36.54 | 2.28 | 25.75 | 1.60 | | | | | 23.58 | 1.47 |
| | HPMC 3mPas | | | 13.92 | 0.87 | | | 8.35 | 0.52 | | | | | 8.35 | 0.52 |
| | HPC | | | | | | | | | | | 5.66 | 0.35 | | |
| | PVA partially hydrolyzed | 23.06 | 1.44 | | | | | | | 19.60 | 1.22 | 16.60 | 1.03 | | |
| | Crosscarmellose Sodium | | | | | | | | | | | | | 2.18 | 0.14 |
| | Polydextrose | | | 10.88 | 0.68 | | | 6.53 | 0.41 | 6.53 | 0.41 | 6.53 | 0.41 | 6.53 | 0.41 |
| | Macrogol 8000 | 11.75 | 0.73 | 2.61 | 0.16 | 3.70 | 0.23 | 1.57 | 0.10 | 9.98 | 0.62 | 8.46 | 0.53 | 1.57 | 0.10 |
| | Talc | 8.70 | 0.54 | 2.18 | 0.14 | 3.26 | 0.20 | 1.31 | 0.08 | 7.40 | 0.46 | 6.26 | 0.39 | 1.31 | 0.08 |
| | | | | | | | | | | | | | | | |
| Active coating | Empagliflozin | 12.50 | 0.78 | 12.50 | 0.78 | 12.50 | 0.78 | 12.50 | 0.78 | 12.50 | 0.78 | 12.50 | 0.78 | 12.50 | 0.78 |
| | HPMC 6mPas | | | 21.51 | 1.34 | 56.46 | 3.52 | 39.79 | 2.48 | | | | | 36.43 | 2.27 |
| | HPMC 3mPas | | | 21.51 | 1.34 | | | 12.90 | 0.80 | | | | | 12.90 | 0.80 |
| | HPC | | | | | | | | | | | 8,74 | 0,54 | | |
| | PVA partially hydrolyzed | 35.62 | 2.22 | | | | | | | 30.28 | 1.89 | 25.65 | 1.60 | | |
| | Crosscarmellose Sodium | | | | | | | | | | | | | 3.36 | 0.21 |
| | Macrogol 3350 | 18.15 | 1.13 | 4.03 | 0.25 | 5.71 | 0.36 | 2.42 | 0.15 | 15.42 | 0.96 | 13.07 | 0.81 | 2.42 | 0.15 |
| | Talc | 13.44 | 0.84 | 3.36 | 0.21 | 5.04 | 0.31 | 2.02 | 0.13 | 11.43 | 0.71 | 9.68 | 0.60 | 2.02 | 0.13 |
| | Polydextrose | | | 16.80 | 1.05 | | | 10.08 | 0.63 | 10.08 | 0.63 | 10.08 | 0.63 | 10.08 | 0.63 |

### Example 4.

Dissolution profile of the tablets of the invention and comparative tablets

Dissolution profiles were tested in a dissolution test according to European Pharmacopoeia 2.9.3 (Apparatus 1 - baskets; 100 rpm; 900 ml; 0.1M HCl, acetate buffer pH 4.5, and phosphate buffer pH 6.8). Dissolution was measured by withdrawing samples at specified time points at 5, 10, 15, 20, 30 and 45 minutes (0 is a start point). Empagliflozin concentration in the samples was determined using HPLC method with UV detection. So called "infinity" level (i.e. reaching 100% dissolution) was determined after increasing the rotation speed of the stirrers to 250 rpm for 15 minutes and analysis of final sample solution.

Generally, for classifying drug product as immediately dissolving (i.e. immediate release drug), the criterion of 85% dissolution in 15 minutes (T85%) is essential to know if complete dissolution is reached before gastric emptying. It is believed that 85% dissolution in 0.1N HCl in 15 minutes can ensure that the bioavailability of the drug is not limited by dissolution. In these cases, the rate limiting step for drug absorption is gastric emptying. Furthermore, where >85% of the drug is dissolved within 15 min, dissolution profiles may be accepted as similar without further mathematical evaluation. This is consistent with The European Medicines Agency recommendations for assessment and evaluation of similarity of the products.

Dissolution of empagliflozin from the tablets of the invention (MEM1A, MEM2A, MEM2B, and MEM2C) was tested in comparison compared with commercial empagliflozin mono product Jardiance^{®} 10 mg and Jardiance^{®} 25 mg in three various dissolution media in Tables 4A, 4B and 4C below.

**Table 4A.**

| | % of dissolution, 0.1M HCl | | | | | | | |
|---|---|---|---|---|---|---|---|---|
| | 0 min | 5 min | 10 min | 15 min | 20 min | 30 min | 45 min | infinity |
| Jardiance^{®} 10 mg | 0.0 | 84.1 | 96.3 | 98.7 | 99.2 | 99.4 | 99.6 | 101.2 |
| Jardiance^{®} 25 mg | 0.0 | 78.0 | 91.4 | 96.2 | 97.9 | 99.0 | 99.2 | 101.1 |
| MEM1A 12.5/1000 mg | 0.0 | 21.8 | 69.5 | 90.7 | 96.8 | 99.1 | 96.9 | 99.4 |
| MEM2A 5/1000 mg | 0.0 | 17.1 | 66.1 | 93.3 | 100.4 | 102.3 | 98.3 | 102.0 |
| MEM2B 12.5/750 mg | 0.0 | 16.3 | 70.5 | 94.8 | 99.1 | 101.1 | 101.6 | 102.0 |
| MEM2C 5/750 mg | 0.0 | 30.7 | 81.4 | 93.1 | 94.9 | 95.7 | 96.1 | 96.6 |

**Table 4B.**

| | % of dissolution, buffer pH 4.5 | | | | | | | |
|---|---|---|---|---|---|---|---|---|
| | 0 min | 5 min | 10 min | 15 min | 20 min | 30 min | 45 min | infinity |
| Jardiance^{®} 10 mg | 0.0 | 87.73 | 99.86 | 101.06 | 101.20 | 101.52 | 101.96 | 103.31 |
| Jardiance^{®} 25 mg | 0.0 | 82.15 | 96.25 | 99.7 | 100.55 | 100.96 | 101.43 | 102.96 |
| MEM1A 12.5/1000 mg | 0.0 | 27.76 | 74.29 | 91.90 | 96.56 | 98.25 | | 98.76 |
| MEM2A 5/1000 mg | 0.0 | 25.72 | 69.97 | 90.21 | 96.64 | 99.13 | | 99.86 |
| MEM2B 12.5/750 mg | 0.0 | 32.61 | 84.96 | 100.75 | 104.17 | 104.89 | | 105.33 |
| MEM2C 5/750 mg | 0.0 | 40.72 | 82.32 | 94.01 | 96.5 | 97.65 | | 98.23 |

**Table 4C.**

| | % of dissolution, buffer pH 6.8 | | | | | | | |
|---|---|---|---|---|---|---|---|---|
| | 0 min | 5 min | 10 min | 15 min | 20 min | 30 min | 45 min | infinity |
| Jardiance^{®} 10 mg | 0.0 | 84.4 | 97.0 | 98.4 | 99.0 | 99.1 | 99.6 | 101.1 |
| Jardiance^{®} 25 mg | 0.0 | 79.9 | 93.3 | 96.6 | 97.5 | 98.1 | 98.5 | 99.9 |
| MEM1A 12.5/1000 mg | 0.0 | 20.6 | 64.8 | 91.9 | 98.9 | 101.8 | | 102.4 |
| MEM2A 5/1000 mg | 0.0 | 26.3 | 72.0 | 90.2 | 94.8 | 96.8 | | 97.4 |
| MEM2B 12.5/750 mg | 0.0 | 31.9 | 80.7 | 95.6 | 98.4 | 99.6 | | 100.0 |
| MEM2C 5/750 mg | 0.0 | 39.9 | 79.1 | 93.3 | 96.4 | 97.7 | | 98.0 |

As can be seen, tablets of the invention in all dosage strengths have desired empagliflozin dissolution profile similar to reference mono empagliflozin product, i.e. more than 85% of empagliflozin is dissolved at 15 minutes from the start, and complete dissolution of whole empagliflozin in time is achieved (so called infinity at the level of about 100%).

Table 4D below shows dissolution of empagliflozin in 0.1M HCl for comparative tablets prepared by the present applicant (MEM13, MEM16, MEM17, and MEM20) having hydroxypropylmethylcellulose as the same film forming material in both intermediate and active coatings, compared to dissolution of commercial empagliflozin mono product Jardiance^{®} 10 mg and Jardiance^{®} 25 mg. Table 4D includes further also dissolution data for prior art tablets Synjardy^{®} 12.5/1000 mg having hydroxypropylmethylcellulose as the same film forming material in both intermediate and active coatings and protected by patents deriving from WO201212040.

**Table 4D.**

| | % of dissolution, 0.1M HCl | | | | | | | |
|---|---|---|---|---|---|---|---|---|
| | 0 min | 5 min | 10 min | 15 min | 20 min | 30 min | 45 min | infinity |
| Jardiance^{®} 10 mg | 0.0 | 84.4 | 97.0 | 98.4 | 99.0 | 99.1 | 99.6 | 101.1 |
| Jardiance^{®} 25 mg | 0.0 | 79.9 | 93.3 | 96.6 | 97.5 | 98.1 | 98.5 | 99.9 |
| MEM13 | 0.0 | 2.6 | 19.3 | 46.3 | 72.9 | 101.3 | 110.8 | 112.4 |
| MEM16 | 0.0 | 1.5 | 17.1 | 40.6 | 63.7 | 96.1 | 109.7 | 111.5 |
| MEM17 | 0.0 | 5.2 | 18.3 | 42.6 | 60.2 | 96.4 | 106.5 | 108.7 |
| MEM20 | 0.0 | 3.4 | 20.6 | 48.4 | 73.9 | 101.9 | 109.9 | 110.8 |
| SynjardyXR^{®} 12.5/1000 mg | 0.0 | 5.7 | 37.0 | 67.0 | 84.0 | 93.8 | | 97.1 |

As can be seen, comparative tablets with hydroxypropylmethylcellulose as a base polymer for both coatings do not provide desired empagliflozin dissolution level, i.e. dissolution of > 85% of empagliflozin at 15 minutes from the start. Even the addition of croscarmellose super-disintegrant to both intermediate and active coating (tablet MEM20) did not improve dissolution at 15 min.

Also commercial market product SynjardyXR^{®} with hydroxypropylmethylcellulose as a base polymer for both coatings does not provide desired empagliflozin dissolution level, i.e. dissolution of > 85% of empagliflozin at 15 minutes from the start.

Table 4E below shows dissolution of empagliflozin in various media for comparative tablets prepared by the present applicant (MEM01, MEM18, and MEM19) having partially hydrolyzed polyvinyl alcohol as the same film forming material in both intermediate and active coatings in comparison with Jardiance^{®} mono products Jardiance^{®} 10 mg and Jardiance^{®} 25 mg.

**Table 4E.**

| | % of dissolution, | | | | | | | |
|---|---|---|---|---|---|---|---|---|
| | 0 min | 5 min | 10 min | 15 min | 20 min | 30 min | 45 min | infinity |
| Jardiance^{®} 10 mg 0.1M HCl | 0.0 | 84.1 | 96.3 | 98.7 | 99.2 | 99.4 | 99.6 | 101.2 |
| Jardiance^{®} 25 mg 0.1M HCl | 0.0 | 78.0 | 91.4 | 96.2 | 97.9 | 99.0 | 99.2 | 101.1 |
| MEM01, 0.1M HCl | 0.0 | 3.90 | 31.30 | 57.40 | 68.67 | 73.20 | 77.73 | 82.90 |
| MEM01, pH 4.5 | 0.0 | 12.60 | 52.17 | 72.67 | 78.03 | 81.90 | 85.,53 | 89.90 |
| MEM01, pH 6.8 | 0.0 | 11.70 | 51.69 | 69.09 | 73.22 | 77.10 | 81.40 | 87.32 |
| MEM18, 0.1M HCl | 0.0 | 17.8 | 57.7 | 77.5 | 80.1 | 85.4 | 87.2 | 91.9 |
| MEM19, 0.1M HCl | 0.0 | 12.1 | 50.8 | 70.0 | 76.4 | 83.4 | 90.3 | 98.2 |

As can be seen, although comparative tablets prepared by the applicant with polyvinyl alcohol as a base polymer for both coatings provided dissolution of > 85% of empagliflozin at 15 minutes from the start, still desired 100% final dissolution (infinity) could not be obtained (tablets MEM01). This was not cured by the addition of polydextrose as a pore-forming agent to both intermediate and active coating (tablets MEM18) nor by the addition of very well soluble hydroxypropylcellulose as an additional film-forming material to polyvinyl alcohol (tablets MEM10).

### Example 5. Stability of the tablets

Stability of the tablets (in terms of empagliflozin stability, i.e. the presence of empagliflozin derived or related substances, further called impurities) was tested in accordance with ICHQ1 standard stability test under accelerated degradation conditions, i.e. at 40°C and relative humidity of 75%.

Empagliflozin containing layer of the tablets was dissolved in the mixture acetonitrile 3: 7 (v/v) and the level of impurities was measured by HPLC method, under following conditions:
HPLC column Synergi, Hydro-RP 80 Å - 250 mm x 4.6 mm, 4.0 µm,
temperature of the column 20°C,
sample temperature 5°C, injection volume 25 µl, flow 1.2 -> 1.0 -> 1.2 ml/min - gradient elution analysis time 47 min,
detection at the wavelength 225 nm
mobile phase A: potassium phosphate (KH₂PO₄) buffer at pH 2.5 +/- 0.05
mobile phase B: acetonitrile
gradient elution as in the table below:

| **Time [min]** | **Flow [ml/min]** | **Mobile phase A [%]** | **Mobile phase B [%]** | **Change** |
|---|---|---|---|---|
| 0 | 1.2 | 75 | 25 | Linear |
| 9 | 1.2 | 65 | 35 | Linear |
| 10 | 1.0 | 65 | 35 | Linear |
| 20 | 1.0 | 65 | 35 | Linear |
| 25 | 1.0 | 48 | 52 | Linear |
| 30 | 1.0 | 30 | 70 | Linear |
| 42 | 1.0 | 30 | 70 | Linear |
| 43 | 1.0 | 75 | 25 | Linear |
| 45 | 1.2 | 75 | 25 | Linear |
| 47 | 1.2 | 75 | 25 | Linear |

The results obtained for tablets of the invention (MEM1A 12.5/1000 mg) and for comparative products with both intermediate and active coating based on partially hydrolyzed polyvinyl alcohol (MEM01 12.5/1000 mg) are presented in Table 5A and Table 5B below, respectively.

Acetylated empagliflozin impurity at RRT 1.94 was detected and identified as monoacetyl empagliflozin (empagliflozin methyl acetate). RRT means relative retention time, and EMPA means empagliflozin. Other unspecified acetyl impurities should appear at RRT 1.89 and 1.96 and were not detected in tested samples.

**Table 5A.**

| Product | MEM1A 12.5/1000 mg | | |
|---|---|---|---|
| Time point | 2 weeks | 1 month | 3 months |
| Impurity RRT vs EMPA | % imp. | % imp. | % imp. |
| EMPA imp. III | nd | nd | nd |
| EMPA Imp. IV | nd | nd | nd |
| EMPA Imp. VI | 0.004 | 0.004 | 0.003 |
| EMPA Imp. V | 0.005 | 0.007 | 0.003 |
| **RRT 1.89** | **nd** | **nd** | **nd** |
| **RRT 1.94** | **nd** | **0.008** | **0.011** |
| **RRT 1.96** | **nd** | **nd** | **nd** |
| RRT 2.06 | 0.019 | 0.019 | 0.020 |
| RRT 2.11 | 0.009 | 0.008 | 0.007 |
| RRT 2.15 | 0.014 | 0.014 | 0.012 |
| RRT 2.23 | 0.018 | 0.014 | 0.015 |
| RRT 2.26 | 0.046 | 0.041 | 0.041 |
| EMPA imp. II | nd | nd | nd |
| Total impurities | < LOQ | < LOQ | < LOQ |

**Table 5B.**

| Product | MEM01 12.5/1000 mg | |
|---|---|---|
| Time point | 2 weeks | 1 month |
| Impurity RRT vs EMPA | % imp. | % imp. |
| EMPA imp. III | nd | nd |
| EMPA Imp. IV | nd | nd |
| EMPA Imp. VI | nd | 0.003 |
| EMPA Imp. V | nd | 0.002 |
| **RRT 1.89** | **nd** | **nd** |
| **RRT 1.94** | **0.006** | **0.022** |
| **RRT 1.96** | **nd** | **nd** |
| RRT 2.02 | 0.017 | 0.020 |
| RRT 2.06 | 0.008 | 0.009 |
| RRT 2.09 | 0.012 | 0.014 |
| RRT 2.16 | 0.014 | 0.016 |
| RRT 2.19 | 0.039 | 0.048 |
| EMPA imp. II | nd | nd |
| Total impurities | < LOQ | 0.05 |

It has been observed that monoacetyl impurity in tested tablets MEM1A appears only as measured at 1 month time point and at the very low level, below the Limit of Detection (LOD) which is 0.017%.

At the same time, if no sugar has been added to coatings compositions based on partially hydrolyzed PVA as a film-forming material (tablets MEM01), acetyl impurity started to appear earlier and at higher level.

This stability enhancing effect is due to the presence of sugar (polydextrose in this example). Apparently, the presence of sugar inhibits or slows down acetylation caused by non-hydrolyzed part of poly vinyl alcohol present in the active coating. This allows to obtain low mass fixed dose combination product metformin hydrochloride plus empagliflozin having desired good dissolution profile despite the presence of acetyl groups in partially hydrolyzed PVA.

## Claims

1. A fixed-dose pharmaceutical composition in the form of a coated tablet for oral administration comprising extended release metformin hydrochloride and immediate release empagliflozin, comprising:
a) an inner extended release tablet core comprising metformin hydrochloride; an extended release polymer and one or more excipients;
b) an intermediate coating on top of the inner core of the tablet, comprising a film-forming material which is hydroxypropylmethylcellulose; and optionally one or two excipients selected from the group consisting of a plasticizer and an anti-tacking material;
c) an active coating on top the intermediate coating, comprising a film-forming material which is partially hydrolyzed polyvinyl alcohol; empagliflozin; and optionally one or two excipients selected from the group consisting of a plasticizer and an anti-tacking material; and
d) optionally, a protective coating on top of the active coating c).

2. The composition according to claim 1, consisting of:
a) an inner extended release tablet core comprising metformin hydrochloride; an extended release polymer and one or more excipients;
b) an intermediate coating on top of the inner core of the tablet, comprising a film-forming material which is hydroxypropylmethylcellulose; and optionally one or two excipients selected from the group consisting of a plasticizer and an anti-tacking material;
c) an active coating on top the intermediate coating, comprising a film-forming material which is partially hydrolyzed polyvinyl alcohol; empagliflozin; and optionally one or two excipients selected from the group consisting of a plasticizer and an anti-tacking material; and
d) optionally, a protective coating on top of the active coating c).

3. The composition according to claim 1 or 2, wherein the active coating c) further comprises a sugar as a stabilizer.

4. The composition according to claim 3, wherein the intermediate coating b) further comprises a sugar as a stabilizer.

5. The composition according to claim 3 or 4, wherein the sugar is polydextrose.

6. The composition according to any one of claims 1 to 5, wherein the intermediate coating b) or the active coating c) or both comprise the plasticizer.

7. The composition according to claim 6, wherein the plasticizer is polyethylene glycol.

8. The composition according to any one of claims 1 to 7, wherein the intermediate coating b) or the active coating c) or both comprise the anti-tacking material, especially talc.

9. The composition according to claim 8, wherein the active coating c) consists of empagliflozin, partially hydrolyzed polyvinyl alcohol, polyethylene glycol, especially polyethylene glycol 3350, talc, and polydextrose.

10. The composition according to claim 8, wherein the intermediate coating b) consists of hydroxypropylmethylcellulose, polyethylene glycol, especially polyethylene glycol 8000, talc, and polydextrose.

11. The composition according to any one of claims 1 to 10, wherein the extended release polymer in the inner extended release tablet core a) is hydroxypropylmethylcellulose, preferably hydroxypropylmethylcellulose K100M.

12. The composition according to any one of claims 1 to 11, wherein said one or more excipients in the inner extended release tablet core a) is a lubricant, preferably magnesium stearate.

13. The composition according to any one of claims 1 to 11, wherein said one or more excipients in the inner extended release tablet core a) is a binder, preferably low-molecular weight hydroxypropylmethylcellulose.

14. The composition according to any one of claims 1 to 11, wherein said one or more excipients in the inner extended release tablet core a) are a lubricant, preferably magnesium stearate, and a binder, preferably low-molecular weight hydroxypropylmethylcellulose.

15. The composition according to any one of claims 11 to 14, wherein the inner extended release tablet core a) consists of metformin hydrochloride, low-molecular weight hydroxypropylmethylcellulose as a binder, hydroxypropylmethylcellulose as the extended release polymer, and magnesium stearate as the lubricant.

16. The composition according to claim any one of claims 1 to 15, wherein the composition comprises the protective coating d), preferably based on polyvinyl alcohol as a film-forming material.

17. The composition according to any one of claims 1 to 16, wherein metformin hydrochloride is present in a unit dosage strength of 750 or 1000.

18. The composition according to any one of claims 1 to 16, wherein empagliflozin is present in a unit dosage strength of 5, 10, 12.5 mg or 25 mg.
